# EUROPEAN PATENT SPECIFICATION

(11) **EP 2 544 624 B1**
(45) Date of publication and mention of the grant of the patent: **23.01.2019**
(21) Application number: 11754211.8
(22) Date of filing: 11.03.2011
(51) Int. Cl.: A61F 2/06, A61M 29/02, A61L 27/18, A61F 2/82, A61L 27/56

(54) **SCAFFOLD SYSTEM TO REPAIR CARDIOVASCULAR CONDITIONS**
GERÜSTSYSTEM ZUR BEHEBUNG KARDIOVASKULÄRER LEIDEN
SYSTÈME D'ÉCHAFAUDAGE POUR LA RÉPARATION D'ÉTATS CARDIOVASCULAIRES

(30) Priority: 11.03.2010 US 312847 P
(43) Date of publication of application: 16.01.2013
(73) Proprietor: Board Of Regents Of the University Of Texas System, Austin, TX 78701 (US)
(72) Inventor: KAUFMANN, J. Jordan Massey, Iowa City, IA 52246 (US); AGRAWAL, Mauli, San Antonio Texas 78231 (US); BAILEY, Steven, San Antonio Texas 78232 (US)
(74) Representative: HGF Limited
(86) International application number: PCT/US2011/028201
(87) International publication number: WO 2011/113001

(56) References cited:
- US-A- 4 329 743
- US-A1- 2003 036 794
- US-A1- 2003 195 611
- US-A1- 2005 009 178
- US-A1- 2005 271 701
- US-A1- 2009 088 828

## Description

### BACKGROUND OF THE INVENTION

### 1. Field of the Invention

The invention generally relates to devices for the treatment of cardiovascular conditions. More specifically, the invention relates to tissue engineering for the treatment of aneurysms or other damaged cardiovascular tissue.

### 2. Description of the Relevant Art

Abdominal aortic aneurysms, commonly referred to as AAA, consist of a 50% enlargement of the abdominal aorta which is believed to be caused by the breakdown of the tunica media, a vessel wall layer primarily composed of smooth muscle cells. While the exact cause of AAA is not well understood, it is believed to be a complex process involving hemodynamic forces as well as local extracellular matrix remodeling, infiltration of macrophages and lymphocytes and increase in matrix metalloproteinase enzymes which all play a role in the destruction of elastin fibers and smooth muscle cells. Over time, a gradual reduction of medial elastin fibers, thinning collagen within the media and thickening of the intima heighten the aneurismal tendency. Loss of elasticity and strength of the tunica media along with compensatory collagen production lead to arterial expansion, forming an aneurysm. Histologically, the aneurysm elastin fragmentation, chronic transmural inflammation and depletion of smooth muscle cells is observed. Aneurysm progression is characterized by molecular mediators and extracellular matrix-degrading proteinases including matrix metalloproteinases 2 and 9. Increased collagen turnover has been targeted as a potential cause of aneurysm growth and rupture.

Studies show that 3% of all individuals aged 50 and over, predominately males, have AAA. In addition, 2.1% of men over 65 years of age will die of ruptured aortic aneurysms. The average aorta at the renal level is approximately 2 cm in diameter; therefore, an aneurysm is technically a 3 cm dilation. By the age of 65, 5% of men and 1.7% of women have an aortic diameter of at least 3 cm. The prevalence of AAA greater than or equal to 3 cm increases 6% with each decade beyond 65 years of age. However, most aneurysms are not considered clinically relevant until they reach 4 cm and surgery is generally not prescribed until they are approximately 5 cm. The risk of rupture is known to increase with the diameter of the aneurysm. Only 25% of patients with ruptured aneurysms reach the hospital and only 10% make it to the operating room. Because of such high mortality rates, it is important to treat the aneurysm before it ruptures.

Current treatment of the AAA includes either open surgery or endovascular aneurysm repair, depending on the patient physiology and pathology. Open surgical treatment of aneurysms was first performed by Dubost and colleagues in 1951 but was reintroduced by Charles Rob in 1963 using the current retroperitoneal approach. With the retroperitoneal approach, the aneurysm is accessed no higher than the 11th rib when the patient is prone. An alternative open surgical method is the transperitoneal technique in which the aneurysm is accessed through an incision along the midline. In 1991, an alternative approach to the open surgical method was introduced by Juan Parodi in which iliofemoral access was used to insert an endovascular graft to cover the aneurysm: endovascular aneurysm repair (EVAR).

EVAR utilizes stent technology to place the graft over the aneurysm and into the iliofemoral arteries, splitting at the bifurcation. The graft serves to block off the aneurismal segment of the aorta without extensive damage to the arteries. Currently FDA approved stent-grafts contain either a woven polyester (PET) or ePTFE graft on a stainless steel, a Cobalt-Chromium alloy, or Nitinol stent. The grafts are fixated using either self-expansion, stents, barbs, or a combination of these. However, because the graft is meant to separate the unhealthy portion from the blood flow, inherent problems exist in the implementation. Tortuosity of the aorta and iliac bifurcation, particularly an angulation of 90° or greater, may lead to an endoleak after implantation in which blood seeps between the graft and the lumen of the aorta, reaching the aneurysm. Calcification and thrombotic events also play a role in limiting EVAR effectiveness, particularly when calcification is greater than 50% or thrombosis is 25%-50%. Success of an EVAR graft is usually defined by the absence of any of the four types of endoleaks. Type I endoleak occurs when blood flows between the graft and the vessel wall at either the proximal or distal ends of the graft. When blood flows into the aneurysm sac from branch vessels, it is considered a Type II endoleak. Type III endoleaks are the result of poor anastomsoes between different sections of the graft. If leakage occurs through the graft material, it is considered a Type IV endoleak. Types II and IV generally resolve spontaneously while Types I and III pose a greater danger and must be repaired during a subsequent procedure.

Testing endovascular grafts for treatment of AAA require first, appropriate cell culture evaluation *in vitro* and structural mechanical properties tests, then an appropriate AAA animal model in order to be properly assessed, particularly in terms of coagulation and fibrinolytic systems. Both canine and swine models are considered appropriate for testing current EVAR devices.

A popular technique used to induce an aneurysm in an animal is the patch model which involves suturing an elliptical patch made of materials such as jejunum, iliac vein, rectus fascia or Dacron into a longitudinal incision made in the aorta. This technique allows gradual enlargement and rupture similar to what is observed in humans. The greatest drawback of this technique is its inconsistency with physiological aneurysm formation and anatomy. More physiologically relevant techniques include mural-stripping, a model in which adventitia and 60-70% of the media are cut away allowing the vessel wall to expand, and the elastase model which uses temporary exposure to an elastolytic agent to break down layers of the vessel wall. However, this technique is difficult to control.
US 2009/088828 A1 relates to a medical device comprising a tubular structure adapted for being implanted in the vasculature of a mammal, the tubular structure being formed, at least in part, of electrically charged nonwoven polymer fibres.
US 2003/195611 A1 relates to a covering and method using electrospinning of very small fibres.

### SUMMARY OF THE INVENTION

A device for treating a cardiovascular condition includes an expandable scaffold comprised of electrospun fibers composed of a biodegradable compound. The scaffold is substantially tubular and comprises a concave surface and a convex surface, wherein the concave surface has a higher concentration of fibers than the convex surface, wherein the concave surface includes fibers with a more looped appearance and the convex surface includes fibers that are more linear; wherein the scaffold is positioned in the vasculature of a mammal to direct tissue development and control blood flow into the undesirable cardiovascular condition. The concave surface of the scaffold may inhibit blood flow and mechanical forces caused by blood flow across the scaffold and vasculature, thereby reducing a rupture of the vasculature while providing an appropriate surface for cell attachment while the less concentrated convex surface facilitates the ingress and organization of cells; and wherein the scaffold is completely degradable over time, leaving new tissue in its place.

In an embodiment, a device for treating a cardiovascular condition includes an expandable scaffold supported by stent technology.

The vascular condition may be an aneurysm, a void, or a semi-void space. The fibers may comprise poly(α-hydroxy esters), or natural polymers, such as Elastin, Collagen, DNA, RNA, Glucosaminoglycans, or mixtures thereof.

The stent may be an expandable stent.

Described herein is a method of treating a vascular condition comprising inserting a device as described above into the vasculature of a mammal; and securing the device in the vasculature.

### BRIEF DESCRIPTION OF THE DRAWINGS

Advantages of the present invention will become apparent to those skilled in the art with the benefit of the following detailed description of embodiments and upon reference to the accompanying drawings in which:
FIG. 1 depicts a schematic diagram of an electrospinner;
FIGS. 2A-2C depict graphs comparing the effect of solution concentration, extrusion rate and voltage on ultimate tensile stress of electrospun tubular scaffolds;
FIGS. 3A-3C depict graphs comparing the effect of solution concentration, extrusion rate and voltage on strain at failure of electrospun tubular scaffolds;
FIG. 4 depicts a graph of the average porosity of scaffolds fabricated using varying parameters;
FIG. 5 depicts SEM images of the contrast between the concave and convex surfaces of a single tubular scaffold representing the gradient of morphological changes throughout the scaffold;
FIGS. 6A-6C depict graphs of the degradation of tubular electrospun scaffolds over 90 days in PBS at 37 °C agitated at 50 RPM (n=6);
FIG. 7 depicts an SEM image of human aortic endothelial cells spread on electrospun tubular scaffold;
FIG. 8 depicts the metabolic activity of human aortic smooth muscle cells in static culture over 14 days on tubular electrospun PCL scaffolds;
FIG. 9 depicts the metabolic activity of human aortic smooth muscle cells in a bioreactor on tubular electrospun scaffolds;
FIG. 10 depicts a graph comparing human aortic smooth muscle cells using different sterilization and seeding techniques;
FIGS. 11A-B depicts SEM images of electrospun scaffolds A (nano) and B (micro) at 2000X;
FIGS. 12A-B depict graphs of change in metabolic activity of hAoEC and hAoSMC in response to scaffolds of different fiber morphology (normalized to day 0 values for each sample);
FIGS. 13A-B depict graphs of cell proliferation over time of hAoEC and hAoSMC on scaffolds composed of either nanofibers (A), microfibers (B) or films (C). Determined using Picogreen to measure dsDNA content, n=6;
FIGS. 14A-D depicts SEM images of electrospun microfibers with human aortic endothelial cells on days 1, 3, 7 and 10;
FIGS. 15A-D depicts SEM images of electrospun microfibers with human aortic smooth muscle cells on days 1, 3, 7 and 10; and
FIGS. 16A-L depicts images of electrospun microfibers with human aortic smooth muscle cells on days 1, 3, 7 and 10.

### DETAILED DESCRIPTION OF THE PREFERRED EMBODIMENTS

EVAR utilizes stent technology to place a graft over the aneurysm from within the blood vessel, essentially blocking off the aneuyrsmal sac from blood flow. Many of the risks associated with EVAR are due to the permanent introduction of a material that is not bioactive. Such risks may be circumvented using a tissue engineering approach to treat AAA. Tissue engineering is a means of rebuilding a tissue by introducing a biodegradable scaffold which is seeded with cells into a defect area. The scaffold provides a three dimensional structure on which the cells can proliferate and organize into a new tissue. Changing the scaffold properties alters the way the cells grow and organize. Taking a tissue engineering approach to treating abdominal aortic aneurysms would allow native cells to infiltrate the scaffold and remodel into an aortic wall of proper diameter.

Applying concepts of tissue engineering, our system uses a highly porous, tubular scaffold placed over the aneurysm endovascularly and seeded naturally by infiltrating cells. This allows for the aneurysm to be "repaved" as the cells secrete extracellular matrix components and organize in response to the scaffold morphology. Infiltrating cells will come from both the blood flowing through the scaffold as well as the surrounding tissue. Initially, the cells act according to the wound healing response. Then the initially adhered cells signal for other more appropriate cells to adhere and migrate through the scaffold.

As different cells adhere, migrate and proliferate a remodeling process takes place in which extracellular matrix components and scaffold fibers are broken down in some areas and bolstered in others. Therefore, as time progresses the scaffold is slowly replaced by functional tissue organized in response to physiological conditions. Eventually the scaffold will be completely degraded leaving tissue in its place of the correct shape and containing vital components such as collagen, elastin and vasa vasorum. At this point the aneurysm will be minimized or no longer present.

By placing the scaffold endovascularly, it is able to reduce the effect of mechanical stimuli while concomitantly providing a structure with high porosity on which appropriate cells can adhere, migrate, proliferate and organize into a new vessel wall. In addition, the infiltration of cells increases the scaffold strength, compliance and integration into the existing tissue. This reduces the chances of endoleaks present by current EVAR stent-grafts. As the vessel wall remodels, the scaffold degrades allowing the new tissue to take over both form and function.

Unlike current EVAR treatments which try to present an impermeable barrier, the scaffold disclosed herein will initially be permeable to allow cell infiltration. Once appropriate cells adhere, put down extracellular matrix components and proliferate, the scaffold will become substantially impermeable. Furthermore, the scaffold is biodegradable, so that as new tissue is formed, our scaffold will slowly be broken down by natural metabolic pathways.

Unlike current tissue engineered blood vessels, the described device may be positioned within the damaged cardiovascular tissue with minimum excision or damage to surrounding tissue.

In an embodiment, scaffolds intended for use in an engineered blood vessel have: a porosity and surface area conducive to cell migration, proliferation and differentiation; stiffness and mechanical strength congruent to native vessels; and a biodegradation rate coinciding with tissue formation. In an embodiment, a scaffold is intended for the aorta and is configured to be implanted endovascularly. A stent for deployment in an aorta is inserted using a catheter in the femoral artery and expanded to the nominal size of the aorta at the aneurysm site. In an embodiment, the scaffold includes a material that can withstand the 5-6x expansion of the stent in the aorta which is necessary for an EVAR procedure. Furthermore the scaffold includes a material that degrades and losses mechanical properties as the tissue is developed allowing the mechanical stresses to gradually be transferred to the new tissue.

The scaffold includes a biodegradable material. In one embodiment, the scaffold consists of nonwoven polycaprolactone (PCL) fibers. PCL is a biodegradable material commonly used in FDA approved clinical applications based on its strength, elastic properties, and extended degradation time. Other polymers which may be used as a scaffold include, but are not limited to, Poly(α-hydroxy esters) such as polylactic acid (PLA), polyglycolic acid (PGA) and poly (D,L-lactide-co-glycolide) (PLGA). These polymers degrade through hydrolysis of their ester bond into acidic monomers which can be removed from the body through normal metabolic pathways thus making them suitable to biodegradation applications. The synthetic nature of PCL makes it more easily tailored for a particular application due to its consistency. Natural polymers such as collagen, elastin or DNA may also be used for this application.

In addition to choosing a feasible material, the scaffold manufacturing process must be appropriate for the given application. Electrospinning is a fiber manufacturing process using electrostatic forces to form nonwoven fibers. A high voltage of one polarity is applied to a polymeric solution or melt, which causes coulombic repulsion as the concentration of positive ions exceeds negative ions. As the solution or melt is expelled and the voltage is applied, the similar charges within the expelled droplet repel each other. The combination of the repulsion within the expelled droplet and the attraction to the collector allows the molecules within the droplet to overcome the surface tension that maintains the droplet form. A jet of solution then accelerates towards the collector, allowing the volatile solvent to evaporate in the distance between the tip of the spinneret and the collector plate. When a fluid is expelled at a sufficient rate and a potential greater than the threshold is applied, the jet is continuous and forms continuous nonwoven fibers ranging from a few nanometers to a few micrometers on the collecting unit. Electrospinning polycaprolactone yields a compliant nonwoven textile well suited for use in aorta scaffolds due to the potential for high porosity and fiber sizes comparable to extracellular matrix components as well as its degradation and mechanical properties. By changing the processing parameters or collecting unit, a myriad of different scaffolds may be formed.

Electrospinning process parameters have a significant effect on the resultant fiber diameter and consistency. In order to prepare a scaffold for use in aneurysm repair, it is desirable to understand how those parameters affect properties of the resultant scaffolds that will play a role in cell proliferation and the success of the scaffold in general. Electrospinning relies on appropriate combinations of a number of parameters including solution concentration, extrusion rate, applied voltage, tip to collector distance, temperature, humidity, volatility of solvent, and polymer characteristics. The effects of these parameters on the properties of electrospun polycaprolactone were studies. To limit the number of variables simultaneously affecting the outcome, the tip to collector distance of the electrospinning device was maintained at 10 cm, and the mandrel rotation was fixed at 587.5 RPM based on preliminary studies. Additionally, polycaprolactone dissolved in chloroform was used as the polymer and environmental conditions within the electrospinning equipment were maintained in the range: 23-24 °C temperature and 45-55% humidity.

In one embodiment, once the scaffold is produced, it is gas plasma treated in order to introduce moieties on the surface that are conducive to cell infiltration and proliferation. Gas-plasma treatment of a scaffold may include subjecting the scaffold to a plasma formed by a reactive gas. A reactive gas may include oxygen, nitrogen, argon, ammonia or combinations thereof.

In one embodiment, the scaffold is treated with chemical stimuli including but not limited to Platelet Derived Growth Facor (PDGF), Vascular Endothelial Growth Factor (VEGF), Angiotensin II (Ang II), Collagen VIII, Collagen I or Collagen V.

A stent system is then used to deploy the scaffold. The scaffold may be attached to a stainless steel, cobalt-chromium alloy, Nitinol, or polymeric stent. The stent scaffold system is implanted using normal EVAR procedures in which a femoral artery is accessed to introduce the system endovascularly then deployed using a balloon catheter. Alternative setups may include spinning the fibers directly onto the stent; altering the polymer used; using a different solvent; or using barbs instead of a stent. Each of these setups would essentially be designed using the same embodiment as the original but would implicate minor changes to the deployment or degradation characteristics of the scaffold system.

After the scaffold system is expanded in the aneurismal aorta, cells from the blood as well as from the native vessel will infiltrate the scaffold as a result of the normal wound healing response. Because the tube is in an expanded form, the fibers will be aligned somewhat concentrically allowing the smooth muscle cells to orient along the same direction, similar to native tunica media while the blood flow will instigate endothelialization with cells oriented in the direction of the flow. Over time, the biomaterial scaffold is hydrolytically degraded and disposed of through natural metabolic pathways leaving new tissue in its place. Because the cells will infiltrate the scaffold, the resulting graft will be directly connected to native tissue thus reducing or eliminating the occurrence of endoleaks unlike current stent-graft systems. In addition, the reinforcement provided by collagen and other extracellular matrix components may contribute to increased stiffness and strength of electrospun scaffolds observed when cells are present. As an added benefit, tissue remodeling may allow collateral vasculature to attach to the new vessel wall, unlike currently used stent grafts.

Investigating interaction of various cells on electrospun fibers, it has been observed that scaffolds made of polymers more resistant to degradation and containing sufficient porosity promote cell integration and proliferation purportedly due to the 3-dimensional structure. This supports the widely held assumption that three-dimensional as opposed to two-dimensional surfaces are preferred by cells over a period of time.

In one embodiment fibers within the scaffold may range in diameter (<200nm to >10µm) and may be arranged to display different porosities (70-85% porous) to accommodate different cell types and attachment tendencies. In addition, the fiber orientation has been noted to play a role in cell adhesion, migration and proliferation. Cells located within arranged fibers frequently display a similar orientation - a characteristic which may be utilized for growing aligned tissues. Investigating cell response to aligned verses nonaligned fibrous scaffolds shows that when fibroblasts were cultured on aligned as opposed to non-aligned polyurethane (PU) fibers, there was an increased amount of collagen produced on the aligned scaffolds, although no increase in cell number was detected. The fiber concentration per area and fiber curviness may alter the cell attachment, proliferation and remodeling. Therefore, the scaffolds are designed to include a morphological gradient from the concave to the convex side. The concave side, includes fibers with a more looped appearance, while the convex side includes fibers that are more linear. This morphological difference may aid in organization of different cell types throughout the scaffold without the need of an additional structure. In addition, the change may aid in reducing blood flow across the scaffold, therefore reducing mechanical force on the aneurysm and reducing the chance of rupture.

Current technology uses more bioinert materials which may result in a fibrous capsule as a result of the immune response. The described embodiments encourage the graft to endothelialize so that it is not rejected (encapsulated). In one embodiment, a scaffold graft, formed as described herein, may utilize the immune response by providing a means for the cells to attach, migrate and proliferate in an organized manner. The gradient comes into play with the cells when the endothelial cells attach to the looped concave surface-- they have more potential points of contact without compromising the porosity. The endothelial cells prefer to grow in a single layer so the concentration of fibers may aid in their attachment and communication. Meanwhile, the convex, more linear, less concentrated side is designed for smooth muscle cells which prefer to organize in striations and follow the length of the fiber. The linearity of the fibers may aid in their organization into circumferential striations. By providing a scaffold designed for cells as opposed to an inert surface, the complications may be decreased. The scaffold grafts described herein may allow for the blood vessels, which supply blood to the aorta, to develop out of necessity. This is, generally, not possible with the current technology which simply blocks off these vessels and potentially leads to burst sacs if one of these is supplying blood to the sac.

To tailor the scaffolds for a particular application, the effects of solution concentration, applied voltage, and extrusion rate on tensile stress and strain, porosity and fiber morphology were examined by changing one of these parameters at a time. After these results were compiled, parameters that yielded scaffolds with unacceptable stress or strain were eliminated and three parameter sets yielding a high, a medium and a low porosity scaffold continued in a degradation study over a 90 day period.

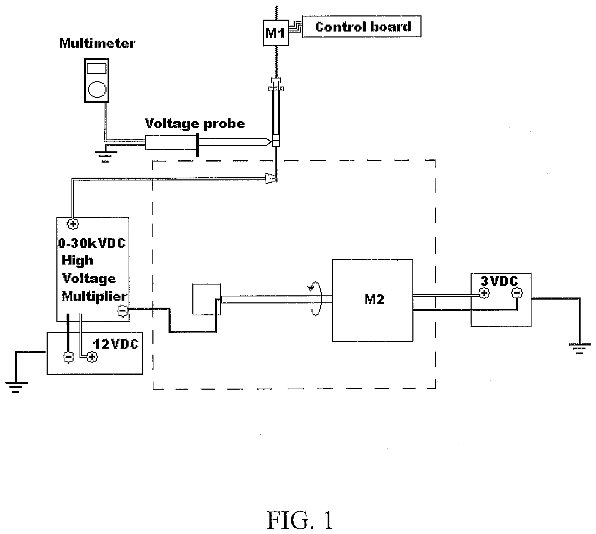

In all our work, the electrospinner used was a custom built model consisting of a 0-30kV voltage source (Information Unlimited) attached to a 22Gs, 2" blunt needle (Hamilton) on a 2.5mL gas tight syringe (Hamilton). An image and schematic of the electrospinner are shown in FIG. 1. The syringe was depressed with a noncaptive bipolar linear actuator (Haydon Switch and Instruments) controlled with a bistep controller (Peter Norberg Consulting, Inc.) using serial commands input through the Hyperlink terminal feature of the PC. In the preliminary work, serial commands of 50r, 125r and 200r were used to define the run rate in microsteps/s/s in order to slew the motor at rates of 16.575 mm/hr, 42.188 mm/hr and 67.5 mm/hr respectively. This produced polymer solution flow rates of 0.012 mL/min, 0.029 mL/min and 0.047 mL/min which are comparable to parameters found in other studies. The positive terminal of the high voltage source was connected via a small alligator clip approximately 3 mm from the tip of the needle. For flat scaffolds, a collecting plate consisting of replaceable aluminum foil over an aluminum screen was connected to the negative terminal of the voltage source and is positioned from the tip of the needle using a screw sensitive to under 1 mm. When tubular scaffolds were made, the aluminum foil and screen were replaced by an aluminum mandrel system. The mandrel was composed of a 0.5 diameter aluminum rod attached to the negative terminal through a bushing. It was turned using a 12 VDC permanent magnet motor (Grainger) which was operated using only 3 VDC to give 587.5 RPM. The spinning area was enclosed by an acrylic case to reduce external interference. Scaffolds were stored in individual vials at room temperature under vacuum at 634.92 mmHg (25 inHg). Both the flat and tubular scaffolds were classified by their manufacturing parameters to determine how these parameters affect mechanical properties. In addition, the effect of the manufacturing parameters on porosity and degradation for the tubular scaffolds was explored.

Electrospinning parameters were optimized to determine which setup provides the best tensile strength and expansion characteristics. After initial testing of a wider range of tip to collector plate distances, solution concentrations and applied voltages, an experiment was setup to examine parameters with the most potential. Samples were made using polycaprolactone (Mn 80000 kDa, Aldrich) dissolved in chloroform (>= 99.8% HPLC grade; Sigma-Aldrich). Concentrations of 8 wt%, 10 wt% and 12 wt% concentrations were used for flat scaffolds while 10 wt%, 12 wt% and 14 wt% solutions were used for tubular scaffolds. Each solution was used within 24 hours and stored in sealed amber bottles between uses. PCL in DCM was examined in early trials with poor results and was thus eliminated from further studies.

For the flat scaffolds, 8kV, 11kV, 14kV and 17kV voltages were applied to each concentration and the syringe was depressed with the 50r serial command corresponding with a 0.012 mL/min flow rate. In addition to a 50r input, the 12 wt% solution was also spun using 125r and 200r commands for the same voltages. This allowed for analyses of the effect of concentration on the resulting scaffolds as well as the effects of voltage and flow rate.

Each flat sample was approximately 0.3 mm thick and cut for mechanical testing using a straight razor blade. The exact thickness and width of each sample was measured by placing the samples between two glasses slides and using calipers to determine the thickness then subtracting the thickness of the slides. This information was used when determining the stress values during mechanical testing. The average fiber diameter, distribution of fiber sizes and sample morphology was analyzed using SEM. For the tubular scaffolds, transverse strips were cut so that the extension axis when tested corresponded with the circumferential stress associated with uniformly expanding the tubular scaffolds. Two straight razor blades were affixed parallel, 0.5 cm apart, allowing consistent strips to be cut without dragging the blade across the samples. Prior to testing, the width and thickness of each strip were measured using an inverted microscope at 40x magnification with Bioquant® software. Ten measurements of each dimension were taken and the average was used to determine an average cross sectional area of each sample. The overall average strip measured 1.1 cm x 0.538 cm x 0.080 cm.

For tensile and elongation testing, ASTM D 5035, Standard Test Method for Breaking Force and Elongation of Textile Fabrics (Strip Method), was followed with some modification due to limitations of scaffold size. Electrospun scaffolds were cut into 20 mm x 10 mm strips and placed in clamps spaced 10 mm apart for a constant rate of extension (CRE) test using an Insight 5 (MTS) system with a 200 lbf load cell. Stress, strain, force, displacement and time were recorded for each strip but only stress and strain were used in analysis due to the variation in sample thickness. The test method was set up to apply a 0.5 N preload to adjust for slack in the samples then the actuator was moved at 1.000 mm/s up to 150 mm. The length of extension was set to exceed the circumference change that would occur when a graft is inserted using a 22F catheter then expanded to 40 mm in diameter.

Data collected from tensile testing of the flat scaffolds showed a clear distinction between lower and higher concentrations in terms of strain. With both 8 and 10 wt% solutions, the scaffolds failed at relatively low strain. However, all 12wt% solutions exceeded tensile stress and strain properties of their lower concentration counterparts. Within the 12wt% group, there was less distinction when comparing expulsion rates and voltages but mid range on both seemed more favorable.

Six flat scaffolds of each parameter were cut into octagons 1.5cm in diameter and three of each were sterilized with oxygen gas plasma while the other three were sterilized with EtO gas. Treatment occurred directly prior to seeding and samples were wetted with Smooth Muscle Growth Supplemented cell media (Medium 231 + SMGS, Cascade Biologics) then incubated for 30 min. Scaffolds were seeded with Human Aortic Smooth Muscle cells (Cascade Biologics, P4) at a density of 2 x 10⁴ cells/cm² using a drop seeding technique. Seeded scaffolds were placed in an incubator and media was changed every other day for 7 days. At day 7, scaffolds were fixed with 4% Formalin then stained with FITC and DAPI. Samples were analyzed using a Leica Fluorescent confocal microscope.

Based on a qualitative assessment of the number of cells per scaffold, gas plasma treated samples made with PCL dissolved in chloroform showed the most promising results. While the sample size was not large enough for statistical significance, the overarching pattern of cell spreading and proliferation on gas plasma treated scaffolds as opposed to EtO scaffolds as well as on scaffolds with larger rather than smaller fibers gives some direction for future studies.

By adjusting electrospinning parameters for flat scaffolds, we observed that while each parameter has an effect on the resulting fibers, the concentration of a solution has a greater impact on fiber morphology than the expulsion rate or the voltage. As the concentration of a solution increased, the diameter of the fiber also increased. However, concentrations higher than 12 wt% displayed two distinct fiber sizes, presumably where a smaller fiber spun off of a larger one due to charge repulsion within the jet as the spinning occurred. Perhaps the most distinct effect of concentration dependence can be observed with the small change in concentration that occurred as solvent evaporated while solution was in the syringe, waiting to be used which created fibers consistent with higher concentrations.

Based on the results from these preliminary studies on flat scaffolds, it was postulated that scaffolds made with a 12 wt% concentration extruded at 0.012 mL/min with 14 kV as well as scaffolds made with 14 wt% concentration extruded at 0.029 mL/min with 12 kV, at a distance of 10 cm will provide sufficient expansion and porosity at the highest tensile strength. In addition, our studies using hASMC support the feasibility of cells prospering on scaffolds made using these conditions. From this data, a more robust study featuring tubular scaffolds was designed and implemented.

As described previously, tubular scaffolds were electrospun from PCL and mechanically tested using a constant rate of extension (CRE) test following ASTM D-5035 "Standard Test Method for Breaking Force and Elongation of Textile Fabrics" as a guideline, although some deviations from the method were necessary due to inherent limitations of the scaffolds. The strip method was used because it is prescribed for nonwoven textiles under the standard although it differs from some currently reported methods which use a dogbone shape. Failure was defined as the point at which the tensile strength became less than or equal to 50% of the ultimate tensile strength. A 889.64N (200 lbf) load cell sending data to Test Works 4 (MTS Systems) was used to calculate stress. Both stress and strain were recorded and graphed from the raw data recorded by Test Works 4. Nine samples of each electrospinning parameter combination were tested (n=9). However, in some cases there was slippage between the specimens and the clamps during testing and these were not included in the analysis.

A pycnometer with a 1.0 cm³ chamber and Helium gas (AccuPyc 1340, Micromeritics) was used to determine the true volume of each tubular scaffold, taking 10 measurements per sample. Bioquant® software was used to measure the nominal volume at 40x magnification on an inverted microscope. For the nominal measurement, samples were sandwiched between two glass slides and an area measurement was taken. Then the samples were stood on end and ten measurements of thickness were taken and averaged. The area was multiplied by the average thickness to determine an average nominal volume. The nominal volume and true volume were used to determine the porosity of the samples. Six samples from each parameter set (n=6) were measured then averaged to determine average porosity for each parameter set. Using scanning electron microscopy (SEM), images were acquired for the various parameters and evaluated for the overall morphology of both the interior and exterior of each sample.

For the degradation study, a high, medium and low porosity scaffold were chosen for analysis from scaffolds considered feasible for aortic aneurysm applications. Aorta scaffolds used with the EVAR technique are introduced into the femoral artery using a catheter. In general, smaller catheter sizes are preferred to reduce damage to the arteries. If a 22F catheter is used, the scaffold circumference will have to expand 5-6 times when it is deployed in the aorta. Because of this demanding high strain capacity during deployment, scaffolds with average strain values less than 550% were considered irrelevant for the degradation study. The scaffold considered to be highly porous has a porosity of 85.4 ± 1.8% (12wt% solution, 0.012 mL/min, 10kV); the medium porosity scaffold is 80.9 ± 1.5% porous (14wt% solution, 0.029 mL/min, 10kV); and the low porosity scaffold is 76.8 ± 5.6% porous (12 wt% solution, 0.029 mL/min, 10kv applied).

A total of 72 scaffolds were made from these three parameter sets (24 scaffolds per set) and were weighed on a microbalance then submerged in 2.0 mL Phosphate Buffered Saline (PBS) in a water bath at a temperature of 37 °C shaking at 50 RPM. After time periods of 1 hour, 30, 60 and 90 days, scaffolds (n=6) corresponding to each parameter set were removed and rinsed three times in deionized water. The scaffolds were then allowed to dry under vacuum for 48 hours at room temperature before being weighed a second time then subjected to mechanical testing as previously described. Results were compared to those of the 1 hour time point which served as control samples to determine trends in mechanical data and changes in weight loss. Care was taken to insure that samples from each time point were tested as quickly as possible and stored under vacuum with desiccant and protected from light between tests.

Parameter sets were compared using one-way ANOVA (α=0.05) to determine significant effects of parameters on stress, strain and porosity as well as degradation. Z-test (α=0.05) and box plots were used to determine outliers within a sample data population.

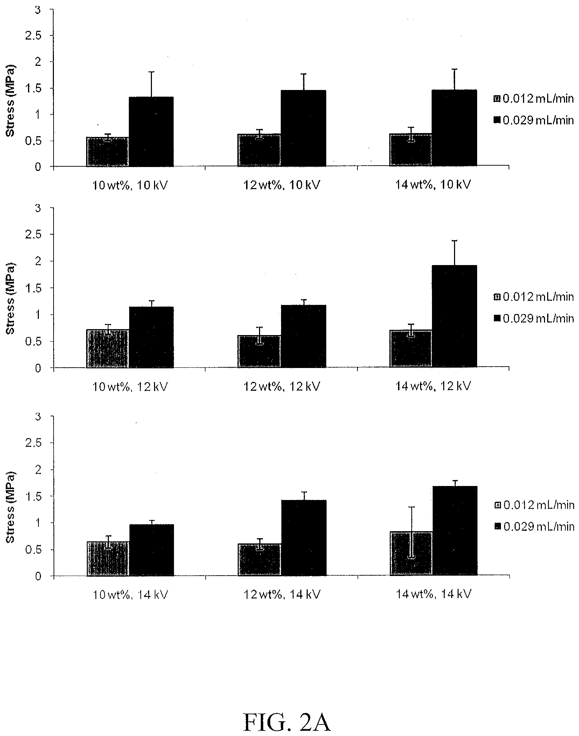

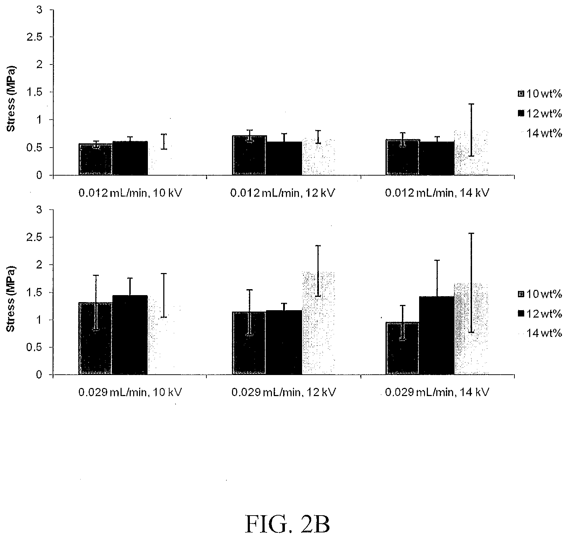

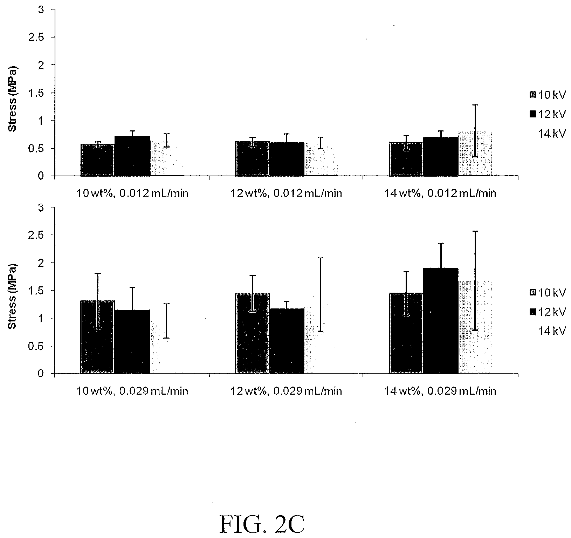

The ultimate tensile strength results from the constant rate of extension test are presented in Figure 2A-2C. While the experimental design called for three separate extrusion rates to be used, all of the samples produced at the 0.047mL/min rate were wet upon reaching the mandrel. This prohibited fiber formation and resulted in a hard, twisted sample incapable of being mechanically tested and inappropriate for use as a tissue scaffold. Therefore, samples spun at the 0.047mL/min rate were eliminated from the study. Some samples spun at 0.029 mL/min exhibited non-uniform collection, occasionally to the extreme of forming a single disc on the mandrel. Although non-uniform, these scaffolds contained well formed fibers and could be tested. Because this was a recurring trend that appears inherent to the set of manufacturing parameters, these samples remained in the study. The 0.012 mL/min rate did not appear to present problems in uniformity on a macroscopic scale. The greatest ultimate tensile strength (UTS) was 1.893 ±0.458 MPa which was associated with the 14wt% solution spun at 0.029 mL/min with 12 kV applied. Out of the nine samples of this configuration tested, 5 slipped out of the clamps and were thus unable to be measured. Comparing UTS based on extrusion rates, all samples showed a significant difference between the slower and faster rate, regardless of the concentration of the solution or the voltage applied. When the UTS was compared based on concentrations, the only significant difference occurred between 12wt% and 14wt% concentrations at 0.029 mL/min when 12kV was applied. Similarly, when comparing UTS based on applied voltage, the only significant difference occurred by applying either 10kV or 12 kV while using the 10wt% concentration at 0.012 mL/min.

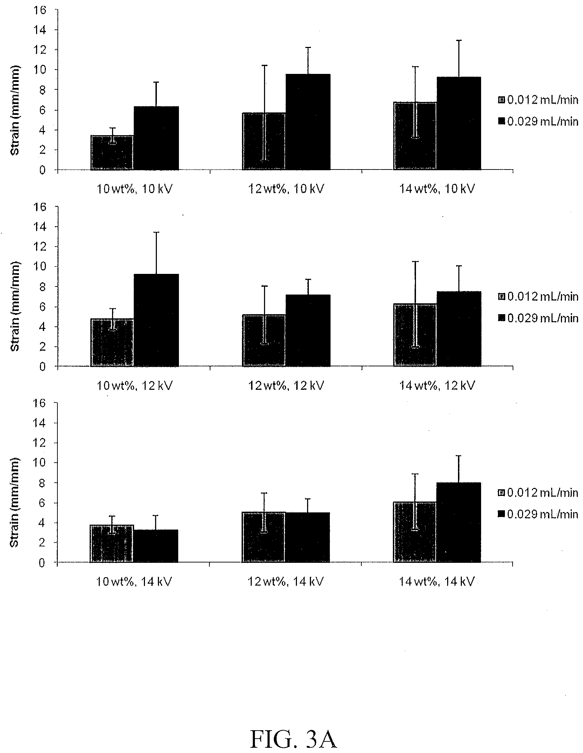

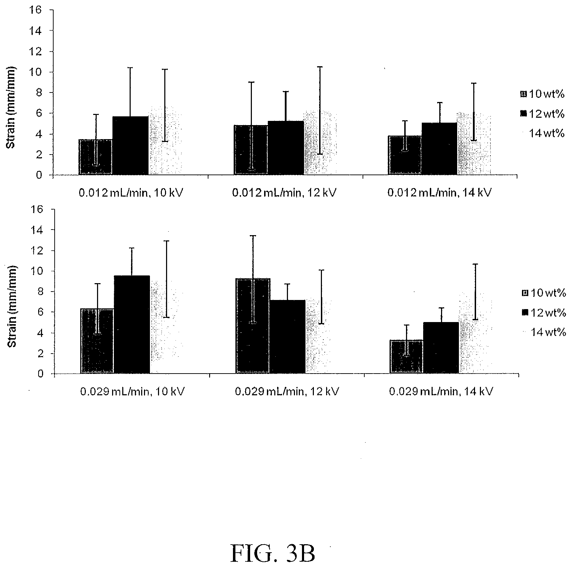

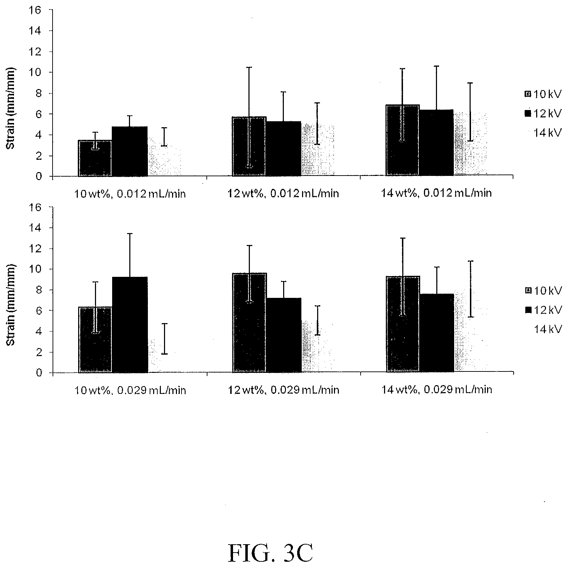

The practical requirements for a device which is inserted in a small vessel then expanded to a large vessel include the strain which can be achieved before failure. Figure 3A-3C demonstrates the average recorded values for strain at failure from the constant rate of extension test. The greatest average strain at failure was recorded at 951.87 ± 272.90% for the sample fabricated using the 12wt% solution extruded at 0.029 mL/min with 10kV applied. When comparing the effect of applied voltage on strain, the strain with 14 kV is significantly less than strain with either 10 kV or 12 kV applied for both 10 wt% and 12 wt% solutions extruded at 0.029 mL/min. However, when the extrusion rate is 0.012 mL/min, the only significant difference occurs with the 10 wt% solution where the samples with 12 kV applied exhibit greater strain at failure than those with 10 kV applied. Examining the effect of concentration on strain at failure, the only significant differences occur between the 10 wt% and 14 wt% as well as between the 12 wt% and 14 wt% when the solution is extruded at 0.029 mL/min with 14 kV applied. Interestingly, unlike the effect extrusion rate has on stress, its effect on strain is minimal. The only significant difference occurs between the 0.012 mL/min and 0.029 mL/min when the 10 wt% solution has either 10 kV or 12 kV applied.

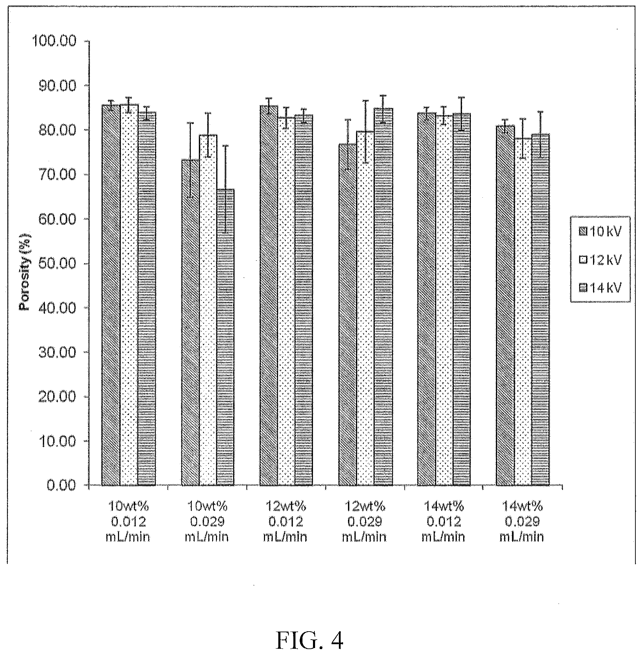

In addition to mechanical requirements, the scaffolds are designed to be favorable for cells. This includes sufficient porosity for cell attachment, migration and proliferation. One of the touted properties of electrospun scaffolds is their fibers resembling extracellular matrix and its porous nature. The average porosity within each sample group remained, for the most part, very similar and with small standard deviations as shown in FIG. 4. The extrusion rate had the greatest effect on porosity as samples made with 10wt% with all applied voltages; 12wt% with 10kV applied; and 14wt% with 10 kV or 12 kV applied showed a significant decrease in porosity when the rate was increased from 0.012 mL/min to 0.029 mL/min. When comparing the effect of solution concentration on porosity, the 10 wt% solution spun at 0.012 mL/min with 12 kV applied was significantly greater than both the 12 wt% and 14 wt% solutions spun with the same configuration. When the extrusion rate was 0.029 mL/min and 14 kV was applied, the scaffolds made with 12wt% solution had significantly greater porosity than those made with the 14wt% solution which were significantly more porous than the 10wt% solution scaffolds. There were, however, no significant differences between scaffold porosity when comparing applied voltage.

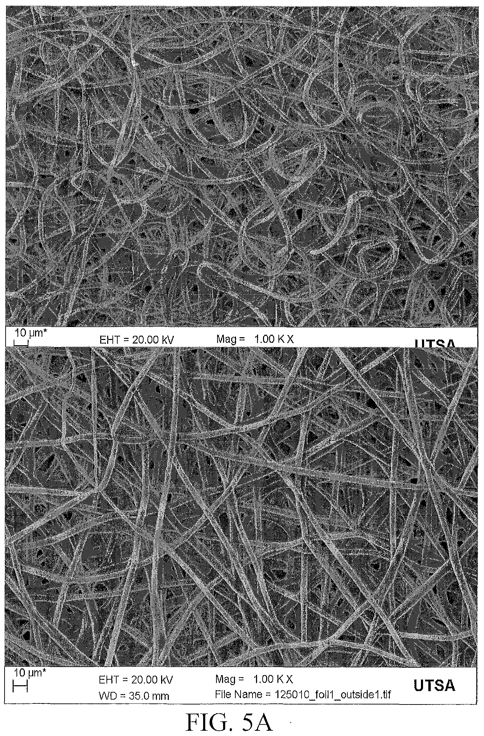

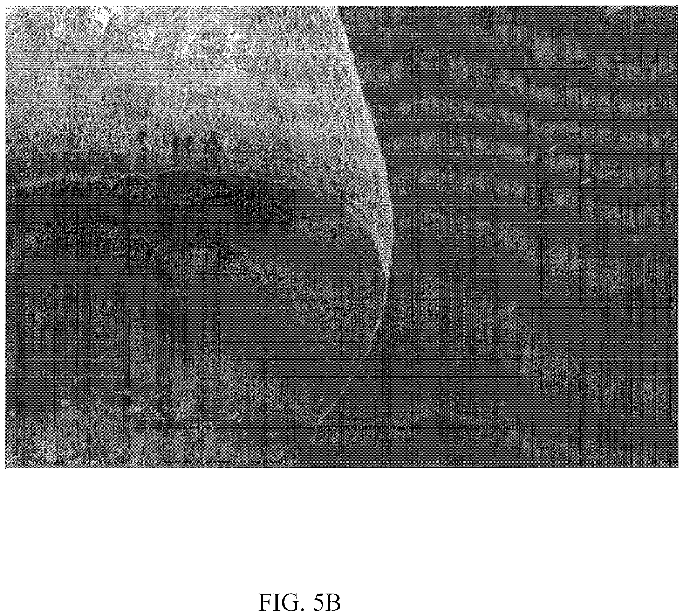

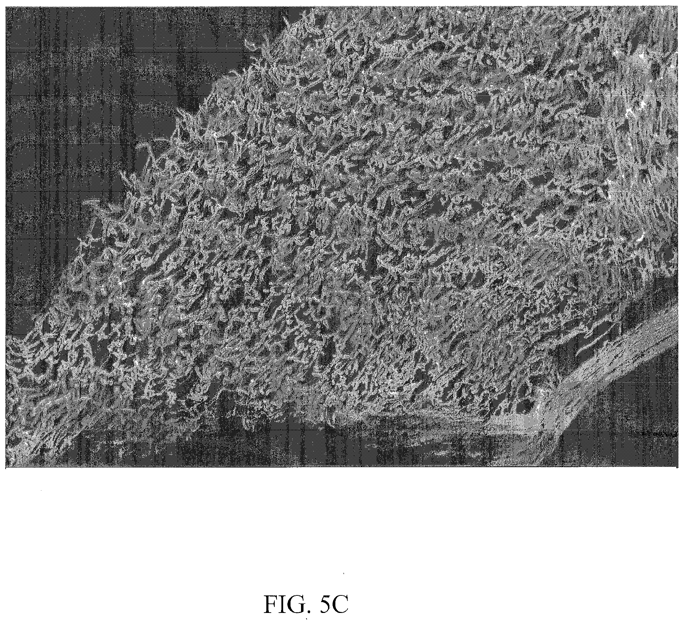

SEM images revealed mild changes in morphology from the interior of the sample to the exterior as shown in FIG. 5. The fibers on the concave side occasionally presented a more curved alignment whereas the fibers on the convex side appeared more linear. While this was not the case for all samples, the 12 wt% concentration appeared to consistently present a greater contrast in linearity between the concave and convex faces while the 10wt% concentration displayed the least contrast.

As a bioresorbable polymer, it is expected that PCL will undergo degradation. However, it is important for the scaffolds to maintain their integrity until viable tissue is formed. It may be expected that scaffolds of higher porosity may lose integrity before scaffolds of lower porosity due to increased surface area.

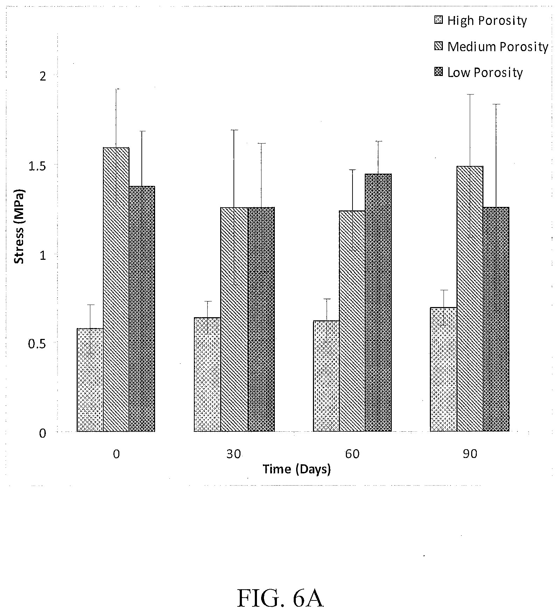

Comparing scaffolds with different porosities over a 90 day time period, there was no significant difference between the ultimate tensile stress from one time point to the initial strength for any of the scaffolds. Results for the tensile stress over time are shown in FIG. 6A and are comparable with values obtained in other areas of this study.

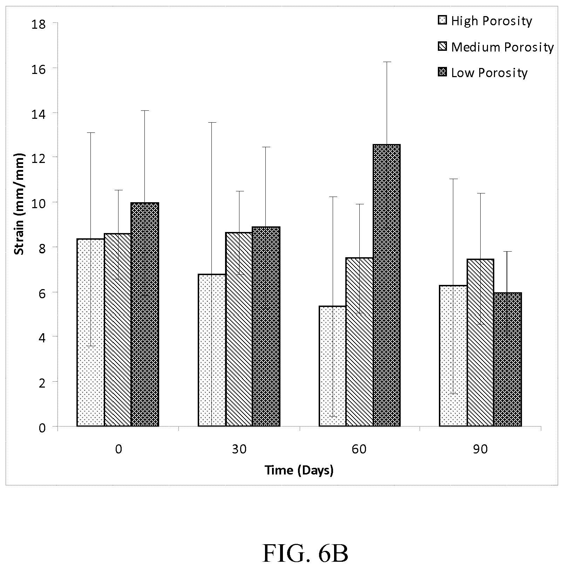

Similarly to results for UTS, there was no significant difference in strain at failure over the 90 day period for any of the scaffolds. FIG. 6B shows a graph of these results.

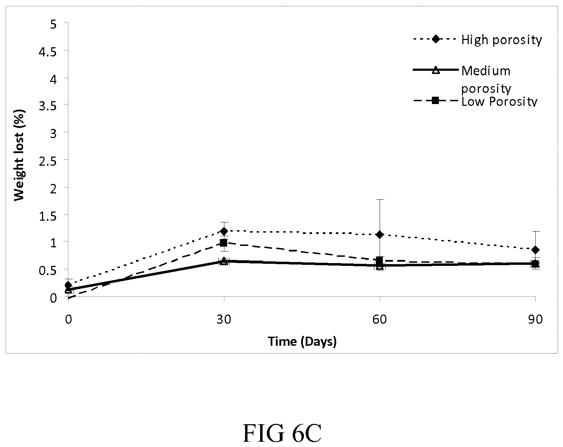

While weight loss over the 90 day time period was observed for all samples as shown in FIG. 6C, it appears to plateau after the initial loss and is minute.

The most definitive effect in this study was the relationship between extrusion rate and ultimate tensile strength. Extrusion rate may have a greater influence on ultimate tensile strength because like the conventional drawing process, as the polymer is extruded, it is drawn and the individual polymer units are aligned to provide greater strength. However, because the voltage component is involved in electrospinning, it provides the mechanism for drawing instead of a mechanical stimulus. The higher extrusion rate appears to result in residual charge buildup as evidenced by the formation of rings on the mandrel at higher rates. This residual charge may be related to increased alignment of polymer units and thus increased ultimate tensile strength.

As noted, the scaffolds made with increased extrusion rates are more likely to form thicker scaffolds on a narrower portion of the mandrel - occasionally leading to a ring formation - whereas the lower extrusion rates tend to form scaffolds that spread out along the mandrel more evenly. The ring phenomenon observed may be related to an extension phenomenon described in polyaniline, an electrically conductive polymer, which allows free movement of electrons. Instead of polyaniline collecting in a flat mat like insulative polymers, the nanofiber network has a tendency to expand in the direction of the applied electric field. This extension is explained as a shortened electron redistribution time causing an accumulation of electric charge at portions of the fiber network which are oriented or bending in a favorable direction.

While the uniform collection is preferred for consistency of the scaffolds and ease of manufacturing, it may be detrimental to the scaffold expansion properties. The scaffolds manufactured at a lower extrusion rate and thus more consistent, presented lower ultimate tensile strength and failed at lower strain values than those made at faster rates. However, the scaffolds that were made at increased extrusion rates presented lower porosity, in general. This may dictate an important compromise to balance the mechanical properties with a preferred porosity for better cell migration and proliferation for an AAA scaffold.

The concave side having more curvy fibers and the convex side having more straight fibers within the same scaffold may contribute to the mechanical properties of the overall scaffold. We also noted that some scaffolds displayed significant necking which led to increased strain. With these scaffolds, they generally broke either by the introduction of elliptical vacancies or by delamination, insinuating that some fibers are breaking before others causing a transfer of tensile forces onto the remaining fibers. On the other hand, some samples had very little necking and broke more abruptly. Samples made at the lower extrusion rate were more likely to break abruptly, occurring in about half of the samples from the parameter set. This may account for the increased deviation within these groups in terms of strain. It was noted, however, that sample sets with large deviations in strain did not show large deviations in stress insinuating that some type of fiber rearrangement is occurring to allow for the expansion and necking but that the fibers themselves have a breaking threshold. This may be related to a gradient of fiber configuration and entanglement throughout the scaffold.

When comparing these properties to the overall trends in porosity, the scaffolds with lower porosity tended to correspond with more consistent concave and convex sides.

Based on the results from the current study, electrospun scaffolds can be classified not only by their manufacturing parameters but also by the morphological characteristics as a whole. For example, the most prominent effect of a manufacturing parameter on mechanical properties is that of extrusion rate on ultimate tensile strength. However, while there is not an equally prominent parameter affecting strain at failure, there are several combinations of parameters which have a significant effect. Ultimately, the entanglement of the scaffold and other morphological properties dictate how the tensile force is distributed and thus influence the strain of the individual scaffolds at failure.

The manufacturing processing parameters - extrusion rate, applied voltage, and solution concentration - can significantly impact the mechanical properties, and morphology of electrospun PCL scaffolds which in turn affect their efficacy as aneurysm repair scaffolds. However, the parameters have less of an effect on the degradation rate of the scaffolds and the corresponding mechanical properties over time. The extrusion rate has the greatest effect on both the ultimate tensile stress and the porosity while playing a lesser role in increasing the strain at failure. Strain at failure appears to rely more on the applied voltage and morphology of the scaffold in general.

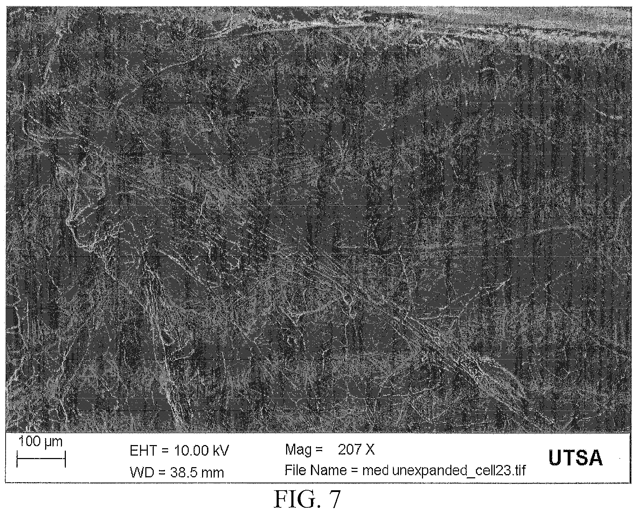

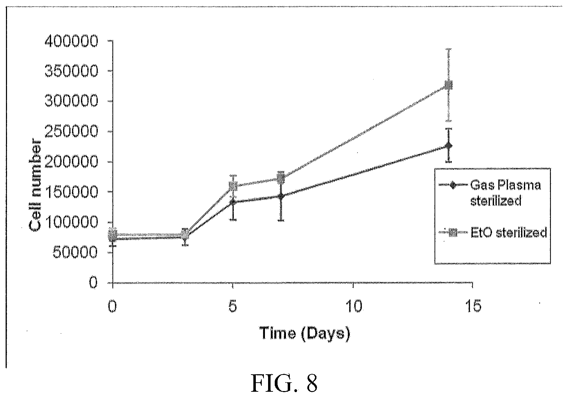

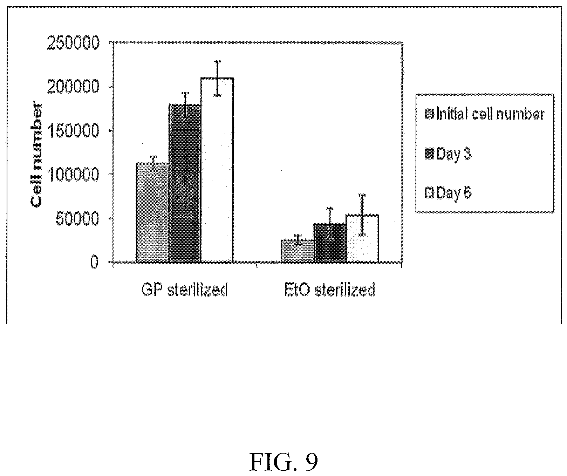

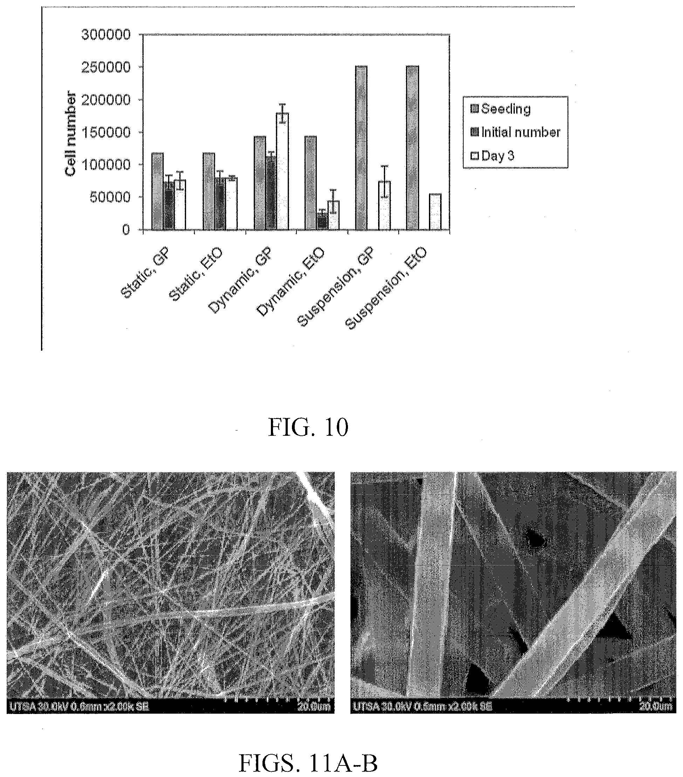

Additional studies were performed to assess cell proliferation on the scaffolds. Tubular scaffolds were placed in both static and dynamic cultures and either human aortic endothelial cells (Cascade Biologics) or human aortic smooth muscle cells (Lonza) were placed on the scaffolds to observe their respective proliferation *in vitro.* FIG.7 shows human aortic endothelial cells spreading on a scaffold when cultured under dynamic flow. While studies with endothelial cells are preliminary, this spreading suggests that the endothelial cells will adhere to the scaffolds and proliferate under dynamic flow. In another study, tubular scaffolds were sterilized with either Ethylene Oxide gas (EtO) (n=3) or Oxygen Gas Plasma (GP) (n=3) then placed in individual well plates and smooth muscle cells were drop-seeded onto the scaffolds. The cells were allowed to proliferate for 14 days, with media changes every other day. The metabolic assay AlamarBlue (Invitrogen) was used to extrapolate cell number at days 0, 3, 5, 7, and 14 as shown in FIG. 8. An increase in cell number indicates that the scaffolds were conducive to cell growth and proliferation. Next, tubular scaffolds were placed in a bioreactor and exposed to a dynamic flow for 5 days with media changes every other day. Scaffolds were once again sterilized with either EtO (n=3) or GP (n=3), seeded with human aortic smooth muscle cells and AlamarBlue was used to measure metabolic activity on days 0, 3 and 5. Results from this study are shown in FIG. 9. The increase in cell number indicates that the cells can proliferate under dynamic flow. While these results are positive it is also important to note whether cells in the fluid passing by the scaffolds will attach. A study was performed in which tubular scaffolds were sterilized with either EtO (n=1) or GP (n=3) and placed in the bioreactor. However, instead of pre-seeding the scaffolds, the cells were placed in suspension in the media that would be perfusing through the system. At day 3, the scaffolds were removed and AlamarBlue was used to determine cell number. The results indicate that the cells are able to adhere to the scaffolds without pre-seeding. FIG. 10 compares the results of the suspension test to the static and dynamic tests in which the cells were pre-seeded. This is an important indication that scaffolds placed in a flow system such as the cardiovascular system will be able to retain cells in the flow thus reducing the need to pre-seed the scaffolds and in turn reducing the time a patient must wait to receive the scaffold.

Studies were conducted to compare different scaffold morphologies. PCL was prepared in three configurations. The first, A, consisted of electrospinning a 9 wt% (e.g., about 8-10 wt%) solution of PCL in 75:25 Chloroform:Methanol (e.g., halogenated organic solvent and alcohol mixture) at 0.035 mL/min with a tip to collector distance of 15 cm and 15 kV applied to the needle of the syringe. The second, B, used electrospinning with a 14 wt% (e.g., about 13-15 wt%) solution of PCL in Chloroform (e.g., a halogenated organic solvent) at 0.029 mL/min extrusion rate, a 10 cm tip to collector distance and 12.0 kV applied. The third set, C, was made from casting 12 wt% (e.g., about 11-13 wt%) PCL solution in Chloroform (e.g., a halogenated organic solvent) on a piece of glass, under a Styrofoam box. After the chloroform evaporated, a film was left which was consistently the same thickness as the B setup, approximately 0.5 mm. The A setup produced thinner scaffolds, approximately 0.3 mm. "C" samples serve as a control to compare the theoretical three-dimensional structure of A and B with a two-dimensional structure. The collector, as mentioned before, consisted of a piece of aluminum foil, shiny side up, which covered an aluminum screen with the negative terminal of the high voltage source applied. After making the scaffolds, they were cut into 5 mm x 5 mm squares using a straight razor blade. SEM was used to image the scaffolds to determine average fiber diameter. FIGS. 11A-B depicts SEM images of electrospun scaffolds A (nano) and B (micro) at 2000X.

In some embodiments, scaffolds were sterilized in open glass scintillation vials by exposing them to high RF oxygen gas plasma for 3 minutes. Scaffolds were grouped for sterilization so that all time points for a group for both cell types were sterilized together to reduce the error that may result in different sterilization within a group. After sterilization, samples may be exposed to sterile cell culture media for their respective cell types in individual wells of ultra-low adhesion well plates.

Human aortic endothelial cells (EC) and human aortic smooth muscle cells (SMC) were purchased from Lifeline cell technologies. The SMC donor was a 49 year old African American male, non-smoker, with hypertension and cardiac disease who died from intracerebral hemorrhage. The EC donor was a 61 year old Caucasian male, non-smoker, with hypertension and cardiac disease who died of intracerebral hemorrhage. SMC were cultured in Invitrogen's basal media, M231, with smooth muscle cell growth supplement and EC were cultured in Lifeline's basal media with Endothelial growth supplement. Both cell types were brought up through P5. Cells were trypsinized, centrifuged, resuspended and counted using a hemacytometer. SMC were introduced to wells with SMC media at a concentration of 4 x 10⁴ cells/scaffold. EC were introduced to wells with EC media at a concentration of 4 x 10⁴ cells/scaffold. Standard curves were also made by seeding a range of volumes of each cell type into a regular well plate. Three scaffolds for each time point were seeded and three replications for the standard curve were seeded. Cells were allowed to attach for 2.5 hours before initial analysis. For metabolic data, this study was replicated 4 times, for proliferation data, the study was replicated twice and for microscopy the study was replicated twice. An n=3 was used for each replication.

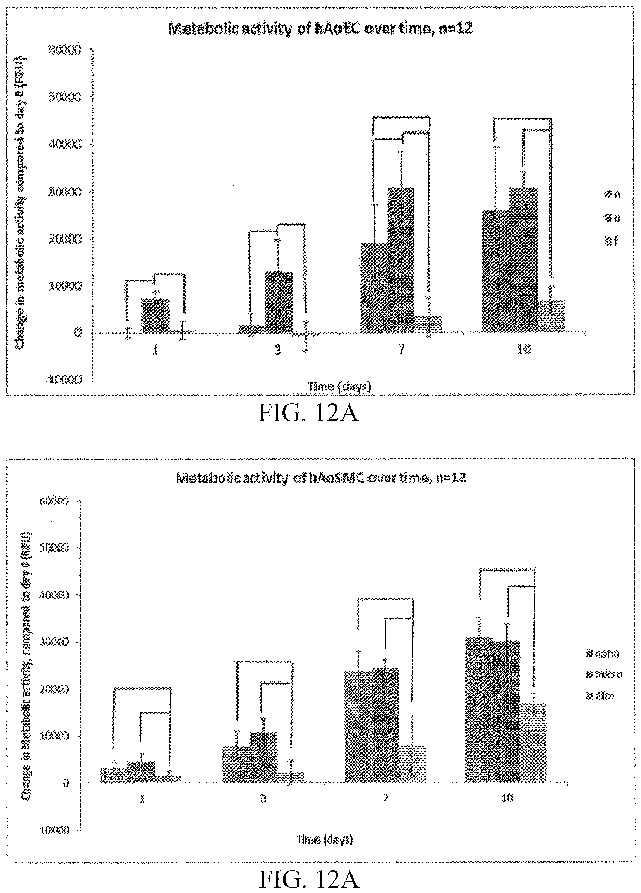

To measure metabolic activity, media was withdrawn from the scaffolds and a 10% alamarBlue (AB) solution in media was added to each well, including the standard curves. The AB solution used the respective media for each cell type. Scaffolds were incubated for 2.5 hours with the AB then the AB was aliquoted in 100 µL volumes into black opaque 96 well plates and read with a fluorescent plate reader at EX:530 EM:590. After AB solution was removed from the wells, scaffolds were rinsed with PBS then plates with day 0 time point scaffolds were wrapped in parafilm and placed in the -80C freezer. Media was replaced in the remaining scaffolds and the plates were placed back in the incubator. This AB process was repeated for days 1, 3, 7 and 10. FIGS. 12A-B depict graphs of change in metabolic activity of hAoEC and hAoSMC in response to scaffolds of different fiber morphology (normalized to day 0 values for each sample).

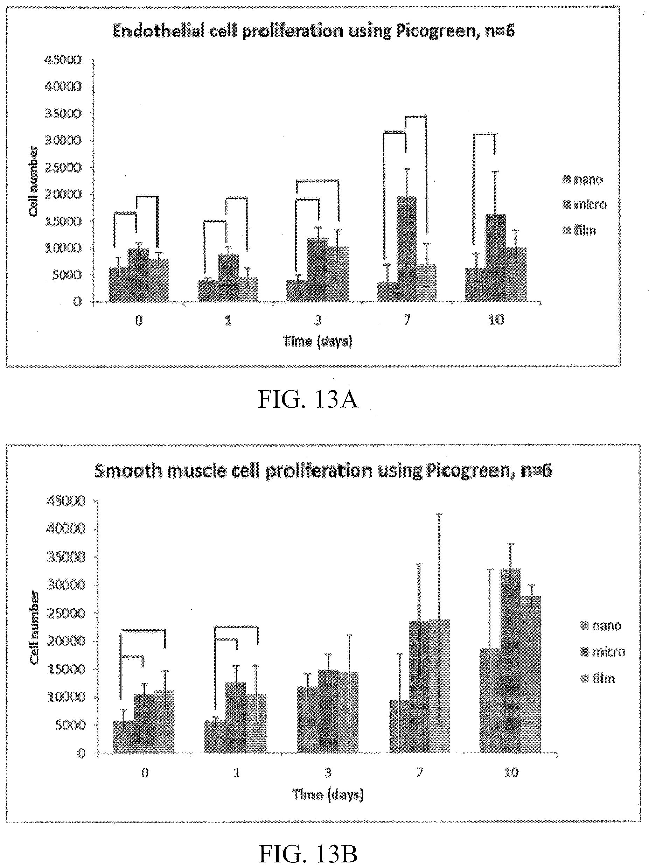

After all time points were completed and frozen, a dsDNA quantification study was performed using Picogreen (PG). Scaffolds were removed from -80 °C and allowed to thaw for 30 min at RT. Proteinase K was diluted in EC media to 1mg/mL and 100 µL was added to each sample and standard curves. The plates were placed in the incubator which was ramped up to 42 °C for 30 min. Plates were removed and placed on a plate shaker for 2 min at #3 intensity. The plates were then placed back in -80 °C and left overnight. The next morning, the plates were removed from the -80 °C and allowed to thaw at room temperature for 30 min. They were once again placed on a plate shaker for 2 min at #3 intensity before being frozen a third time at -80 °C for another 30 min then thawed at room temperature for 30 min. 500 µL of TE buffer was added to all of the Plate 1 samples. Then 5 replicates of 100 µL each was removed to a DNAse and RNAse-free 96 well plate. Plates 2 and 3 were placed in -20 °C freezer. The PG assay solution was mixed and consisted of 100 µL PG with 21 mL of TE buffer. 100 µL of PG solution was added to the well plates so that the total volume per well was 200 µL. The plates were allowed to incubate a few minutes in the dark then read with a fluorescence plate reader at EX:485 EM:528. The same technique was repeated for plates 2 and 3. FIGS. 13A-B depict graphs of cell proliferation over time of hAoEC and hAoSMC on scaffolds composed of either nanofibers (A), microfibers (B) or films (C). Determined using Picogreen to measure dsDNA content, n=6.

Scanning electron microscopy was used to image both fibrous scaffolds before the introduction of cells as well as at each time point. When cells were present, the samples were fixed in 4% Paraformaldehyde, then dehydrated using an ethanol gradient before being placed in a vacuum oven at room temperature.

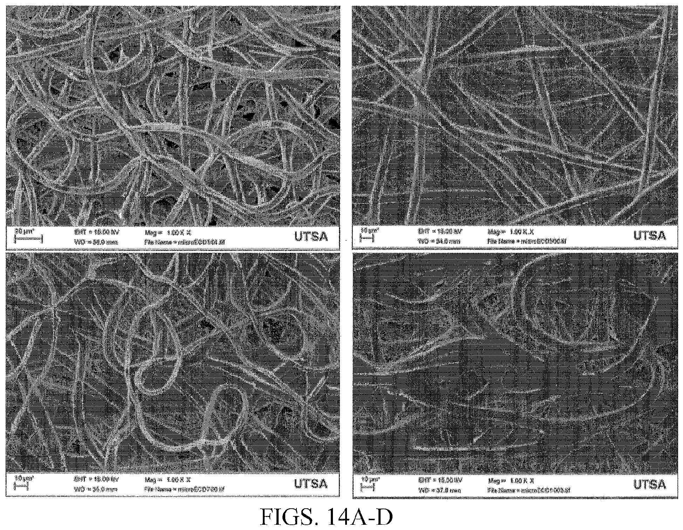

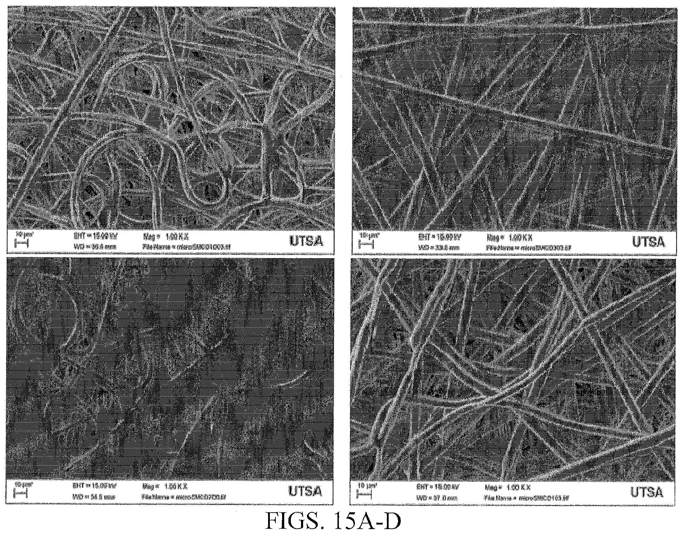

Samples for each time point were fixed in 4% paraformaldehyde then stained with either α-actin conjugated FITC or anti-CD-31 with a fluoraphor and DAPI to stain the nuclei. The samples were mounted in Slowfade then observed with a confocal fluorescence microscope using their respective wavelengths. FIGS. 14A-D depicts SEM images of electrospun microfibers with human aortic endothelial cells on days 1, 3, 7 and 10. FIGS. 15A-D depicts SEM images of electrospun microfibers with human aortic smooth muscle cells on days 1, 3, 7 and 10.

One-way ANOVA was used to determine a significant increase in cell number and metabolic activity. Tukey test was used Post hoc. A z test was used to determine outliers.

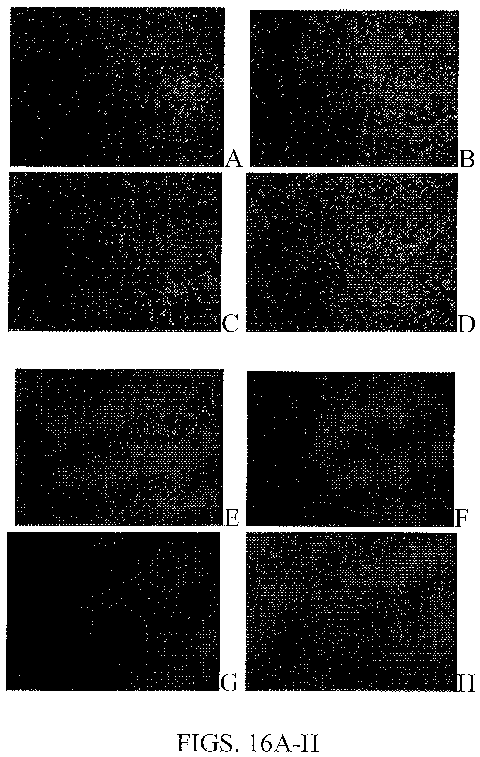

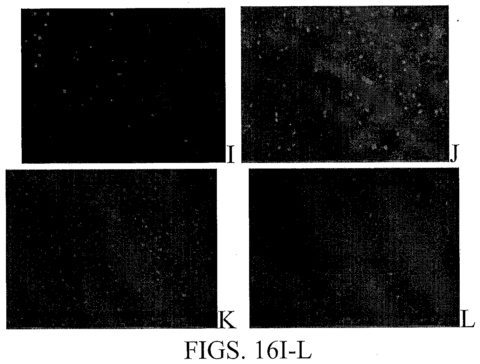

FIGS. 16A-L depicts images of electrospun microfibers with human aortic smooth muscle cells on days 1, 3, 7 and 10 (FIGS. 16A-D depict SMC on scaffold A, FIGS. 16E-H depict SMC on scaffold B, and FIGS. 16I-L depict SMC on scaffold C each set for respective days 1, 3, 7 and 10). The scaffolds are shown to be significantly different although they are manufactured from the same material using similar techniques. The "A" scaffolds are measured to be 0.245 µm ± 0.158 whereas "B" scaffolds are 6.744 µm ±0.265. Based on both the metabolic and proliferation data, it can be determined that endothelial cells respond more positively to microfibers than either films or nanofiber scaffolds made of the same material. More specifically, it should be noted that on the nanofibers, the endothelial cells show increased metabolism but not increased proliferation suggesting that the cells may be distressed. A similar trend is observed on the film controls but not on the microfiber scaffolds. The contrast of metabolic activity as well as proliferation with visual images for microfiber scaffolds suggests that the cells have infiltrated the scaffolds, unlike the other samples.

## Claims

1. A device for treating a cardiovascular condition comprising:
an expandable scaffold comprised of electrospun nonwoven biodegradable fibers and wherein the scaffold is substantially tubular and comprises a concave surface and a convex surface and wherein the concave surface has a higher concentration of fibers than the convex surface;
wherein the concave surface includes fibers with a more looped appearance and the convex surface includes fibers that are more linear;
wherein, when the scaffold is positioned in the vasculature of a mammal to direct tissue development and control blood flow into the undesirable cardiovascular condition, the concave surface of the scaffold may inhibit blood flow and mechanical forces caused by blood flow across the scaffold and vasculature, thereby reducing a rupture of the vasculature while providing an appropriate surface for cell attachment while the less concentrated convex surface facilitates the ingress and organization of cells; and
wherein the scaffold is completely degradable over time leaving new tissue in its place.

2. The device of claim 1, wherein the cardiovascular condition is an aneurysm.

3. The device of any one of the above claims, wherein the cardiovascular condition is a void or semi-void space.

4. The device of any one of the above claims, wherein the fibers comprise poly (a-hydroxy esters).

5. The device of any one of the above claims, wherein the fibers comprise natural polymers.

6. The device of any one of the above claims, wherein the fibers comprise Elastin, Collagen, DNA, RNA, Glucosaminoglycans, or mixtures thereof.

7. The device of any one of the above claims, wherein the scaffold is composed of a nonwoven textile.

8. The device of any one of the above claims, wherein the fibers comprise polycapro lactone.

9. The device of any one of the above claims, wherein the scaffold is comprised of nonwoven micro fibers electrospun from a biomaterial compound.

10. The device of claim 1, wherein the scaffold is supported by at least a portion of a medical device.

11. The device of claim 10, wherein the medical device is a stent.

## Patentansprüche

1. Vorrichtung zum Behandeln eines kardiovaskulären Zustands, umfassend:
ein expandierbares Gerüst, das aus elektrogesponnenen nichtgewebten bioabbaubaren Fasern besteht, wobei das Gerüst im Wesentlichen rohrförmig ist und eine konkave Oberfläche und eine konvexe Oberfläche umfasst und wobei die konkave Oberfläche eine höhere Konzentration von Fasern als die konvexe Oberfläche aufweist;
wobei die konkave Oberfläche Fasern mit einem mehr schleifenartigen Aussehen umfasst und die konvexe Oberfläche Fasern umfasst, die mehr linear sind;
wobei, wenn das Gerüst in dem Gefäßsystem eines Säugers angeordnet ist, um Gewebeentwicklung zu lenken und Blutfluss in den unerwünschten kardiovaskulären Zustand zu beherrschen, die konkave Oberfläche des Gerüsts Blutfluss und mechanische Kräfte, die von Blutfluss über das Gerüst und das Gefäßsystem verursacht werden, hemmen und dadurch Reißen des Gefäßsystems verringern kann, während eine geeignete Oberfläche zur Zellanhaftung bereitgestellt wird, während die weniger konzentrierte konvexe Oberfläche das Eintreten und Organisieren von Zellen erleichtert; und
wobei das Gerüst im Zeitverlauf vollständig abbaubar ist, um neues Gewebe an seiner Stelle zu belassen.

2. Vorrichtung gemäß Anspruch 1, wobei der kardiovaskuläre Zustand ein Aneurysma ist.

3. Vorrichtung gemäß einem der vorstehenden Ansprüche, wobei der kardiovaskuläre Zustand ein Hohlraum oder halbhohler Raum ist.

4. Vorrichtung gemäß einem der vorstehenden Ansprüche, wobei die Fasern Poly(α-hydroxyester) umfassen.

5. Vorrichtung gemäß einem der vorstehenden Ansprüche, wobei die Fasern natürliche Polymere umfassen.

6. Vorrichtung gemäß einem der vorstehenden Ansprüche, wobei die Fasern Elastin, Collagen, DNA, RNA, Glucosaminoglycane oder Gemische davon umfassen.

7. Vorrichtung gemäß einem der vorstehenden Ansprüche, wobei das Gerüst aus einem nichtgewebten Textil besteht.

8. Vorrichtung gemäß einem der vorstehenden Ansprüche, wobei die Fasern Polycaprolacton umfassen.

9. Vorrichtung gemäß einem der vorstehenden Ansprüche, wobei das Gerüst aus nichtgewebten Mikrofasern besteht, die aus einer Biomaterialverbindung elektrogesponnen sind.

10. Vorrichtung gemäß Anspruch 1, wobei das Gerüst von wenigstens einem Teil einer medizinischen Vorrichtung getragen wird.

11. Vorrichtung gemäß Anspruch 10, wobei die medizinische Vorrichtung ein Stent ist.

## Revendications

1. Dispositif de traitement d'un état cardiovasculaire, comprenant :
un échafaudage expansible constitué de fibres biodégradables non tissées électrofilées, et dans lequel l'échafaudage est sensiblement tubulaire et comprend une surface concave et une surface convexe, et dans lequel la sur face concave a une concentration plus élevée de fibres que la surface convexe ;
dans lequel la surface concave comprend des fibres ayant une apparence plus bouclée et la surface convexe comprend des fibres qui sont plus linéaires ;
dans lequel, lorsque l'échafaudage est positionné dans le système vasculaire d'un mammifère pour diriger le développement tissulaire et contrôler la circulation sanguine qui parvient dans l'état cardiovasculaire indésirable, la surface concave de l'échafaudage peut inhiber la circulation sanguine et les forces mécaniques produites par la circulation sanguine à travers l'échafaudage et le système vasculaire, en réduisant ainsi une rupture du système vasculaire tout en apportant une surface appropriée pour la fixation de cellules, tandis que la surface convexe moins concentrée facilite la pénétration et l'organisation de cellules ; et
dans lequel l'échafaudage est capable de se dégrader progressivement dans sa totalité en laissant place à de nouveaux tissus.

2. Dispositif selon la revendication 1, dans lequel l'état cardiovasculaire est un anévrisme.

3. Dispositif selon l'une quelconque des revendications précédentes, dans lequel l'état cardiovasculaire est un espace vide ou semi-vide.

4. Dispositif selon l'une quelconque des revendications précédentes, dans lequel les fibres comprennent des poly(α-hydroxyesters).

5. Dispositif selon l'une quelconque des revendications précédentes, dans lequel les fibres comprennent des polymères naturels.

6. Dispositif selon l'une quelconque des revendications précédentes, dans lequel les fibres comprennent de l'élastine, du collagène, de l'ADN, de l'ARN, des glucosaminoglycanes, ou des mélanges de ceux-ci.

7. Dispositif selon l'une quelconque des revendications précédentes, dans lequel l'échafaudage se compose d'un textile non tissé.

8. Dispositif selon l'une quelconque des revendications précédentes, dans lequel les fibres comprennent de la polycaprolactone.

9. Dispositif selon l'une quelconque des revendications précédentes, dans lequel l'échafaudage est constitué de microfibres non tissées électrofilées à partir d'un composé qui est un biomatériau.

10. Dispositif selon la revendication 1, dans lequel l'échafaudage est supporté par au moins une partie d'un dispositif médical.

11. Dispositif selon la revendication 10, le dispositif médical étant un stent.
